# EUROPEAN PATENT APPLICATION

(11) **EP 4 019 034 A1**
(43) Date of publication of application: **29.06.2022**
(21) Application number: 20306687.3
(22) Date of filing: 23.12.2020
(51) Int. Cl.: A61K 38/10, A61P 9/10

(54) **PEPTIDES FOR PREVENTING OR TREATING HEART AND BLOOD VESSEL DISEASES**

(71) Applicant: RD Nephrologie, 34090 Montpellier (FR)
(72) Inventor: JANKOWSKI, Joachim., D-52159 ROETGEN (DE); ZHOUJUN, Wu., D-52072 AACHEN (DE); ORTH-ALAMPOUR, Setareh., D-50668 KÖLN (DE); ARGILÉS, Àngel, 34980 SAINT-GÉLY-DU-FESC (FR); GAYRARD, Nathalie., 34090 MONTPELLIER (FR)
(74) Representative: IPAZ

(57) **Abstract**

The present invention pertains to the field of peptides for their use as a medicament, in particular for the prevention and the treatment of heart and blood vessel disease, more particularly of heart and blood vessel diseases associated with vascular calcification. A peptide according to the invention comprises, or consists of, an amino acid sequence of at least 3 amino acids from the Calcium Binding Factor (CBF).

SEQUENCE LISTING

<110>  RD-Nephrology

<120>  Peptides for preventing or treating heart and blood vessel
       diseases

<130>  IOBM20RDNCBF

<160>  13

<170>  PatentIn version 3.5

<210>  1
<211>  19
<212>  PRT
<213>  Homo sapiens

<400>  1

Leu Glu Gly Gln Glu Glu Glu Glu Asp Asn Arg Asp Ser Ser Met Lys
1               5                   10                  15

Leu Ser Phe

<210>  2
<211>  10
<212>  PRT
<213>  Homo sapiens

<400>  2

Leu Glu Gly Gln Glu Glu Glu Glu Asp Asn
1               5                   10

<210>  3
<211>  10
<212>  PRT
<213>  Homo sapiens

<400>  3

Glu Glu Glu Asp Asn Arg Asp Ser Ser Met
1               5                   10

<210>  4
<211>  9
<212>  PRT
<213>  Homo sapiens

<400>  4

Arg Asp Ser Ser Met Lys Leu Ser Phe
1               5

<210>  5
<211>  5

<212>  PRT
<213>  Homo sapiens

<400>  5

Leu Glu Gly Gln Glu
1               5


<210>  6
<211>  5
<212>  PRT
<213>  Homo sapiens

<400>  6

Glu Glu Glu Asp Asn
1               5


<210>  7
<211>  4
<212>  PRT
<213>  Homo sapiens

<400>  7

Leu Glu Gly Gln
1


<210>  8
<211>  4
<212>  PRT
<213>  Homo sapiens

<400>  8

Glu Gly Gln Glu
1


<210>  9
<211>  4
<212>  PRT
<213>  Homo sapiens

<400>  9

Gly Gln Glu Glu
1


<210>  10
<211>  4
<212>  PRT
<213>  Homo sapiens

<400>  10

Gln Glu Glu Glu
1

<210>  11
<211>  4
<212>  PRT
<213>  Homo sapiens

<400>  11

Glu Glu Glu Glu
1


<210>  12
<211>  4
<212>  PRT
<213>  Homo sapiens

<400>  12

Glu Glu Glu Asp
1


<210>  13
<211>  4
<212>  PRT
<213>  Homo sapiens

<400>  13

Glu Glu Asp Asn
1

## Description

### FIELD OF THE INVENTION

The present invention relates to the field of prevention and the treatment of heart and blood vessel diseases, more particularly of heart and blood vessel diseases in connection with vascular calcification. The invention relates to the use of peptides, peptide analogs and compositions comprising said peptides or peptide analogs, for their use as medicaments for the prevention or the treatment of heart and blood vessel diseases or to ameliorate one or more symptoms of tissue calcification, and to treat diseases comprising a symptom of vascular calcification component.

### TECHNICAL BACKGROUND

Heart and blood vessel diseases represent a public health problem, and are a risk factor of many age-related diseases. Vascular calcification occurs more frequently in the case of calcium and/or phosphate metabolic impairment, with one of the major associated risk factor being hypertension, and it may be observed in patients suffering from kidney insufficiency.

There is a wide variety of treatment options for heart and vessel diseases, such as regulation of blood pressure, regulation of dyslipidemia, and stent implantation. However, there is none preventing or treating calcification and calcification related - heart and vascular diseases. Vascular calcification is *in-vivo* regulated by biomolecules like inhibiting properties such as pyrophosphate and Fibroblast-Growth Factor-23 (FGF-23). However, a pharmacological use of these substances is not possible.

Therefore, there is still a need for a treatment of heart and blood vessel pathologies in connection with vascular calcification.

The thesis from Silvia Salem ("Isolation and identification of unknown vascular remodelling biomolecules from bovine adrenal glands" Freie Universitat Berlin, 2012) discloses the isolation from adrenal gland, identification and amino acid sequence of two peptides respectively designated as Calcification Blocking Factor (CBF) and Vasoconstriction Inhibiting Factor (VIF).

The protein chromogranin A (Accession number NP 001266.1 for chromogranin-A isoform 1 preproprotein [Homo sapiens] in GenPept database, July 10, 2020) is expressed in secretory cells of different tissues of neuronal, neuro-endocrine, and endocrine origin (Helle, K.B. et al., "The endocrine role for chromogranin A: a prohormone for peptides with regulatory properties." Cell Mol Life Sci 64, 2863-2886 (2007)). This protein is associated with cardiovascular (patho)physiology (Vaingankar, S.M., et al. « Effects of chromogranin A deficiency and excess in vivo: biphasic blood pressure and catecholamine responses". J Hypertens 28, 817-825 (2010).

The inventors have demonstrated, *in vitro, ex vivo* and *in vivo,* that the peptide designated as "Calcium-Blocking Factor" (CBF) (SEQ ID N°1) and fragments of CBF, governs calcium handling and prevents vascular calcification. CBF, and fragments of CBF, are shown to reduce calcium content of cells and aortic rings in calcifying cultures and from aortas from VDN (Vitamin D3 Nicotine) animals. Pulse pressure as a marker of arterial stiffness of VDN animals treated with CBF, or fragments of CBF, significantly decreased. The calcification inhibitory effect of some fragments of CBF is at least as active as the full 19 amino acids CBF peptide.

The inventors have also shown that CBF is enzymatically cleaved from chromogranin A by calpain 1 and kallikrein and is secreted into the plasma by the adrenal glands. CBF concentration has been observed to be significantly decreased in patients with CKD stage 5, known to have vascular calcifications. Also, CBF prevents vascular smooth muscle cell (VSMC) transdifferentiation into osteoblast-like cells within the vascular wall via sodium-dependent phosphate transporter PIT-1 and by decreasing the gene expression of BMP2 followed by the key transcription factors OSX, RUNX2 and MSX2. In addition, CBF reduces the phosphorylation of SMAD1 and -5, suggesting interference in the BMP2 pathway.

Without being bound by any particular theory, among possible mechanisms for the action of peptides according to the invention, CBF and CBF fragments are susceptible to govern calcium handling and prevent vascular calcification by preventing vascular smooth muscle cell (VSMC) transdifferentiation into osteoblast-like cells within the vascular wall via sodium-dependent phosphate transporter "PIT-1" and by decreasing the gene expression of BMP2 and/or interfering in the BMP2 pathway.

The inventors also demonstrated that CBF is a mediator modulating the differentiation of vascular smooth muscle cells and, hence, vascular calcification. This adrenal gland-derived compound may enhance our understanding of pathophysiological conditions, such as calcific arteriopathy. Since CBF is secreted by adrenal granules and is present in effective concentrations in human plasma, CBF is likely to participate in the regulation of vascular calcification processes and, hence, in reducing its harmful consequences.

Therefore, peptides comprising, or consisting of, CBF or CBF fragments amino acid sequences represent drug candidates for preventing and/or treating heart and blood vessel diseases, in particular, heart and blood vessel diseases associated with vascular tissue calcification. These peptides present the advantage of being easily synthesized, due to their short size.

### SUMMARY OF THE INVENTION

In a first aspect, the present invention relates to a peptide comprising, or consisting of, a sequence of at least 3 amino acids from SEQ ID N°2, for its use as a medicament. More particularly, the invention relates to a peptide comprising, or consisting of, a sequence of at least 3 amino acids from SEQ ID N°2, for its use as a medicament for the prevention and/or the treatment of heart and blood vessel disease. Even more particularly, the present invention relates to a peptide comprising, or consisting of, a sequence of at least 3 amino acids from SEQ ID N°2, for its use as a medicament for the prevention and/or the treatment of heart and blood vessel disease associated with vascular calcification.

According to the invention, SEQ ID N°2 consists, from its N-terminal to its C-terminal sequence, of the amino acids: LEGQE EEEDN, and is also designated as "CBF1" in the present patent application. The amino acid sequences SEQ ID N°2 corresponds to amino acids N°1 to N°10, from the N-terminal to the C-terminal extremity, of the amino acid sequence of the Calcium Binding Factor ("CBF").

In a second aspect, the present invention relates to an isolated nucleic acid sequence comprising, or consisting of, a nucleotide sequence encoding for a peptide according to the invention, for its use as a medicament. According to a particular embodiment, the invention relates to an isolated nucleic acid sequence comprising, or consisting of, a nucleotide sequence encoding for a peptide according to the invention for its use as a medicament for the prevention or the treatment of heart and blood vessel disease, and particularly of heart and blood vessel disease associated with vascular calcification.

In a third aspect, the present invention relates to a composition comprising:
- at least a peptide according to the invention for its use as a medicament, and/or
- at least an isolated nucleic acid sequence encoding for said peptide.

According to a preferred embodiment of this third aspect, a composition according to the invention is a pharmaceutical composition.

In a fourth aspect, the present invention relates to the use of a peptide comprising, or consisting of, a sequence of at least 3 amino acids from SEQ ID N°2, for the prevention or the treatment of heart and blood vessel disease, particularly for the prevention or the treatment of a heart and blood vessel disease associated with vascular calcification.

In a fifth aspect, the present invention relates to a method for the prevention or the treatment, particularly for the prevention or the treatment of a heart and blood vessel disease, more particularly to a method for the prevention or the treatment of a heart and blood vessel disease associated with vascular calcification, wherein said method comprises the administration of a peptide comprising, or consisting of, a sequence of at least 3 amino acids from SEQ ID N°2, and/or the administration of an isolated nucleic acid encoding for a peptide comprising, or consisting of, a sequence of at least 3 amino acids from SEQ ID N°2.

### DETAILED DESCRIPTION

According to a first aspect, the present invention relates to a peptide comprising, or consisting of, a sequence of at least 3 amino acids from SEQ ID N°2, for its use as a medicament.

The inventors have identified and functionally characterized several different peptides, which amino acid sequences share at least a sequence of 3 amino acids from SEQ ID N°2.

These peptide sequences have been shown to present a significant *in vitro, ex vivo* and *in vivo* activity in appropriate animal models. Therefore, they represent promising candidates for their use as a medicament.

According to the invention, by "peptide", it is preferably intended an isolated peptide, wherein by "isolated peptide" it is intended a peptide obtained by a method known by a person skilled in the art, including chemical synthesis and recombinant production, said peptide being subsequently separated, by purification or partial purification, from its initial environment.

By "amino acid", abbreviated as AA, it is intended a carboxylic acid also including a functional amine group. In the sense of the present invention, such an amino acid can be a natural or a non-natural amino acid. Said amino acid can be of D or L form. The different isomers of a peptide according to the invention are included in the scope of the present invention.

By "amino acid sequence" it is intended a sequence of amino acids from its N-terminal to its C-terminal. It is also intended a sequence of amino acids possibly comprising peptide analogs.

Each amino acid is herein represented according to the IUPAC amino-acid abbreviation; such as follows:

| Amino acid | Three letters code | One letter code |
|---|---|---|
| Alanine | Ala | A |
| Arginine | Arg | R |
| Asparagine | Asn | N |
| Aspartic acid (Aspartate) | Asp | D |
| Cysteine | Cys | C |
| Glutamine | Gln | Q |
| Glutamic acid (Glutamate) | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophane | Trp | W |
| Tyrosine | Tyr | Y |
| Valine | Val | V |

By "medicament" it is intended a "drug", which is understood as a pharmacology active compound of a peptide which sequence comprises, or consists in, at least 3 amino acids from SEQ ID N°2, and a "prodrug", which is understood as a pharmacodynamically inert compound comprising a peptide which sequence comprises, or consists in, a sequence of at least 3 amino acids from SEQ ID N°2, wherein said prodrug is capable of being transformed in vitro and/or in vivo into a pharmacologically active drug as defined above. A prodrug may comprise, for example, a peptide according to the invention in association with a protective group, the presence of such a protective group being intended to facilitate the passage of the compounds according to the invention through a biological membrane, in particular the membrane of an epithelium or a cellular membrane, after the *in vivo* administration and before the entry into the target cell. The nature of such a protective group thus influences the lipophilicity of the compound and its degradation kinetics.

According to a particular embodiment, the present invention relates to a peptide comprising, or consisting of, a sequence of at least 3, at least 4, at least 5, at least 6, at least 7, at least 8, at least 9 or 10 amino acids from SEQ ID N°2 for its use as a medicament.

In a particular embodiment, a peptide according to the invention comprises, or consists of a sequence of at least 3, at least 4, at least 5, at least 6, at least 7, at least 8, at least 9 or 10 amino acids from SEQ ID N°2 for its use as a medicament for the prevention or the treatment of heart and blood vessel disease.

By "heart and blood vessel diseases" it is intended pathologies involving blood vessels (vascular diseases) and/or heart (cardiac diseases). Examples of said pathologies include: coronary artery disease, peripheral arterial disease, cerebrovascular disease, renal artery stenosis, aortic aneurysm, cardiomyopathy, hypertensive heart disease, heart failure, pulmonary heart disease, cardiac dysrhythmias and valvular heart disease.

In a more particular embodiment of this first aspect, a peptide according to the invention comprises, or consists of a sequence of at least 3, at least 4, at least 5, at least 6, at least 7, at least 8, at least 9 or 10 amino acids from SEQ ID N°2 for its use as a medicament for the prevention or the treatment of heart and blood vessel disease associated with vascular calcification.

By "vascular calcification" it is intended the presence of deposits of calcium-phosphate crystals in vessel walls; wherein "vascular calcification" may extend to "cardiac valvular calcification" and to small and large vessels calcification. Clinical consequences of vascular calcification are due to effects of modification of the structure of the arteries. They include, in particular, a loss of elasticity, an increase arterial stiffness, an increase in arterial pressure, or hypertension, with its consequences such as cerebrovascular accidents, decreased blood flow to target organs, leading to mesenteric ischemia, hypoxia of the lower limbs under stress (intermittent claudication syndrome), coronary hypoxia (angina and myocardial infarction), dermal necrosis, calciphylaxis and other pathologies.

In a particular embodiment, the present invention relates to a peptide which consists in a sequence of 3, 4, 5, 6, 7, 8, 9 or 10 amino acids from SEQ ID N°2, for its use as a medicament.

In another particular embodiment, the present invention relates to a peptide for its use as a medicament, wherein the amino acid sequence of said peptide comprises a sequence of 3, 4, 5, 6, 7, 8, 9 or 10 amino acids from SEQ ID N°2, in combination with other amino acids and/or with any vector, carrier or drug delivery system. A person skilled in the art can select appropriate peptide delivery system or vector, according to its target. According to this embodiment, said combination preferably takes the form of a covalent linkage. A person skilled in the art can select appropriate peptide delivery system or vector, according to its target

In a more particular aspect, the present invention relates to a peptide comprising, or consisting of, a sequence of at least 4 amino acids from SEQ ID N°2, which have shown a significant activity. Their respective amino acids sequences are summarized in Table 1.

**Table 1**

| Reference | Amino acid sequence | Reference to CBF |
|---|---|---|
| SEQ ID N°1 | LEGQE EEEDN RDSSM KLSF | CBF |
| SEQ ID N°2 | LEGQE EEEDN | "CBF1", amino acids 1-10 of CBF, |
| SEQ ID N°5 | LEGQE | amino acids 1-5 of CBF |
| SEQ ID N°6 | EEEDN | amino acids 6-10 of CBF |
| SEQ ID N°7 | LEGQ | amino acids 1-4 of CBF |
| SEQ ID N°8 | EGQE | amino acids 2-5 of CBF |
| SEQ ID N°9 | GQEE | amino acids 3-6 of CBF |
| SEQ ID N°10 | QEEE | amino acids 4-7 of CBF |
| SEQ ID N°11 | EEEE | amino acids 5-8 of CBF |
| SEQ ID N°12 | EEED | amino acids 6-9 of CBF |
| SEQ ID N°13 | EEDN | amino acids 7-10 of CBF |

In a preferred embodiment of this first aspect, a peptide according to the invention comprises a sequence of 3, 4, 5, 6, 7, 8, 9 or 10 amino acids from SEQ ID N°2, for its use as a medicament for the prevention and/or the treatment of heart and blood vessel disease.

More preferably, a peptide according to the invention comprises a sequence of 4, 5, 6, 7, 8, 9 or 10 amino acids from SEQ ID N°2, with the exception of a peptide having an amino acid sequence of SEQ ID N°1 (LEGQE EEEDN RDSSM KLSF) for its use as a medicament for the prevention and/or the treatment of heart and blood vessel disease.

More preferably, a peptide according to the invention comprises a sequence of 4, 5, 6, 7, 8, 9 or 10 amino acids from SEQ ID N°2, with the exception of a peptide having an amino acid sequence of SEQ ID N°3 (EEEDN RDSSM) for its use as a medicament for the prevention and/or the treatment of heart and blood vessel disease.

Even more preferably, a peptide according to the invention comprises a sequence of 4, 5, 6, 7, 8, 9 or 10 amino acids from SEQ ID N°2, wherein said sequence is chosen among the following: SEQ ID N°2, SEQ ID N°5, SEQ ID N°6, SEQ ID N°7, SEQ ID N°8, SEQ ID N°9, SEQ ID N°10, SEQ ID N°11, SEQ ID N°12 and SEQ ID N°13.

A peptide according to the invention can be obtained by any conventional method known in the art. In particular embodiment, a peptide according to the invention is produced by synthesis, more particularly by chemical synthesis wherein synthesis steps are well known to the person skilled in the art and use commercially available and inexpensive compounds. In another particular embodiment, a peptide of the invention is obtained by recombinant production, using a nucleic acid sequence encoding for said peptide, which is integrated in an appropriate expression system, and with steps well known by a skilled person in the art.

In a particular embodiment, the present invention relates to a chemically synthesized peptide comprising, or consisting of, a sequence of at least 3 amino acids from SEQ ID N°2, for its use as a medicament for the prevention and/or the treatment of heart and blood vessel disease.

In another particular embodiment, the present invention relates to a peptide comprising, or consisting of, a sequence of at least 3 amino acids from SEQ ID N°2, for its use as a medicament for the prevention and/or the treatment of heart and blood vessel disease in connection with vascular calcification.

In another particular embodiment, the present invention relates to a peptide comprising, or consisting of, a sequence of at least 3 amino acids from SEQ ID N°2, for its use as a medicament for the prevention and/or the treatment of vascular calcification.

In a particular embodiment, the present invention relates to a peptide comprising, or consisting of, a sequence of at least 3 amino acids from SEQ ID N°2, for its use as a medicament for the prevention and/or the treatment of heart and blood vessel disease for a patient in need thereof.

According to the invention, by "patient", it is intended a human or animal patient.

More particularly, the present invention relates to a peptide comprising, or consisting of, a sequence of at least 3 amino acids from SEQ ID N°2, for its use as a medicament for the prevention and/or the treatment of heart and blood vessel disease, for a patient in need thereof, said patient being chosen among: elder patients, patients suffering from a metabolic disease, patients suffering from a calcium and/or phosphate metabolic disease and/or patients suffering from kidney disease.

In a second aspect, the present invention relates to an isolated nucleic acid sequence comprising, or consisting of, a nucleotide sequence encoding for a peptide according to the invention. A person skilled in the art of developing a medicament comprising, or consisting of, an isolated nucleic acid sequence encoding for a defined peptide is able to determine the appropriate nucleic acid sequence able to lead to the production of a peptide of interest; said person skilled in the art is also able to select the appropriate nucleic acid vector, including an expression vector. The present invention also relates to a vector comprising a nucleotide sequence encoding for a peptide according to the invention; the present invention also relates to a host comprising a vector comprising a nucleotide sequence encoding for a peptide according to the invention.

In a third aspect, the present invention relates to a composition comprising a peptide according to the invention for its use as a medicament for the prevention and/or the treatment of heart and blood vessel disease, or an isolated nucleic acid sequence encoding for said peptide of the invention. According to a preferred embodiment of this third aspect, a composition according to the invention is a pharmaceutical composition.

A pharmaceutical composition according to the invention comprises at least a peptide according to the invention, in an effective amount, for its use as a medicament for the prevention and/or the treatment of heart and blood vessel disease, and at least one pharmaceutically acceptable excipient.

According to the present invention, the term "effective amount" of a composition means the amount, which is sufficient to allow for preventing or treating the disease in the subject, particularly. It is understood that the effective dosage will be dependent upon the age, sex, health, and weight of the recipient, the nature of the disease or condition being investigated, and the nature of the effect desired.

The effective amount can be tailored to the individual subject, as is understood and determinable by one of skill in the art, without undue experimentation.

By "pharmaceutically acceptable" it is designated any ingredient, which is useful in the preparation of a pharmaceutical composition and which is generally safe, nontoxic and not undesirable biologically or otherwise, and which is acceptable for veterinary use or for use in humans. By "pharmaceutically acceptable excipient" is understood any substance other than the active ingredient, which is intended to confer a consistency, a flavor, a color to a medicament, while avoiding any interaction with the active ingredient.

The pharmaceutically acceptable excipient according to the invention will be selected, depending on the pharmaceutical form and the desired route of administration, from the usual excipients, which are known to the person skilled in the art, with a view to being administered to humans or to animals.

The routes of administration, the dosages and the optimal galenic forms of a pharmaceutical composition according to the invention can be determined according to the criteria generally taken into consideration in the determination of a pharmaceutical treatment suited to a subject, such as, for example, the age or the body weight of the patient, the severity of his/her general condition, the tolerance to the treatment, the side effects observed. Depending on the desired administration type, the pharmaceutical composition according to the invention can moreover include at least one pharmaceutically acceptable excipient. The pharmaceutical composition according to the present invention can moreover include at least one pharmaceutical adjuvant known to the person skilled in the art, selected from the thickeners, the preservatives, the perfumes, the dyes, the chemical or mineral filters, the hydration agents, mineral water, etc.

In a particular embodiment, a pharmaceutical composition according to the invention takes the form of a solution, an aqueous suspension or in a dry condition. More particularly, a pharmaceutical composition according to the invention takes the form of tablets, pills, capsules or powders.

In a particular embodiment, a pharmaceutical composition according to the invention takes the form susceptible to be administered via a topical route such as cutaneous, transcutaneous or percutaneous, oral, parenteral, nasal, or intravenous.

According to a particular aspect, a composition according to the invention comprises a mixture of at least two peptides according to the invention.

In a fourth aspect, the present invention relates to the use of at least one peptide comprising, or consisting of, a sequence of at least 3 amino acids from SEQ ID N°2, for the prevention or the treatment of heart and blood vessel disease, preferably for the prevention or the treatment of a disease associated with vascular calcification.

In a particular embodiment, said at least one peptide comprising, or consisting of, a sequence of at least 3 amino acids from SEQ ID N°2, is used in combination with any medical or surgical treatment.

In a fifth aspect, the present invention relates to a method for the prevention or the treatment of heart and blood vessel disease, preferably to a method for the prevention or the treatment of a disease associated with vascular calcification.

A method for prevention and/or treatment of a disease of heart and blood vessel includes the administration to a subject, who has a need for a compound according to the invention.

In a particular embodiment, a method of treatment according to the invention comprises the administration of at least one peptide comprising, or consisting of, a sequence of at least 3 amino acids from SEQ ID N°2, in combination with any medical or surgical treatment.

Characteristics, advantages and uses of the subject-matter of the present invention are more detailed hereunder, in an illustrated and non-limiting way.

The following figures and examples are provided herein for purposes of illustration only and are not intended to be limiting unless otherwise specified.

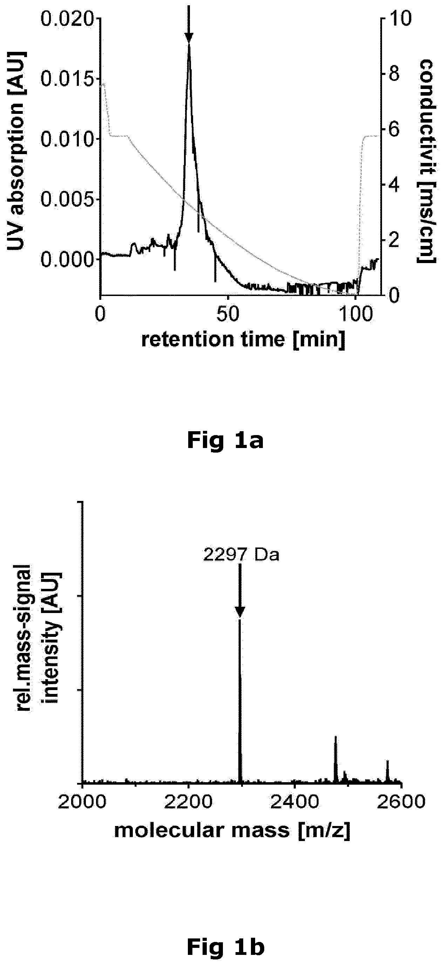

Figures 1a and 1b represent the main steps of identification and characterisation of *Calcium Blocking Factor* (CBF), as described in example 1. Figure 1a represents a reversed-phase chromatogram of the adrenal extract, the pooled fractions were tested in a calcification assay for inhibitory effects on the calcification of aortic rings (AU: arbitrary units). Figure 1b represents the MALDI-TOF/TOF mass-spectrometry of the fraction shown in Figure 1a to identify the sequence of the inhibitory factor of calcification. The mass-signal (m/z) at 2,297 Da (M+H⁺) corresponds to the peptide sequence of SEQ ID N°1 also designated as "calcification blocking factor" (CBF).

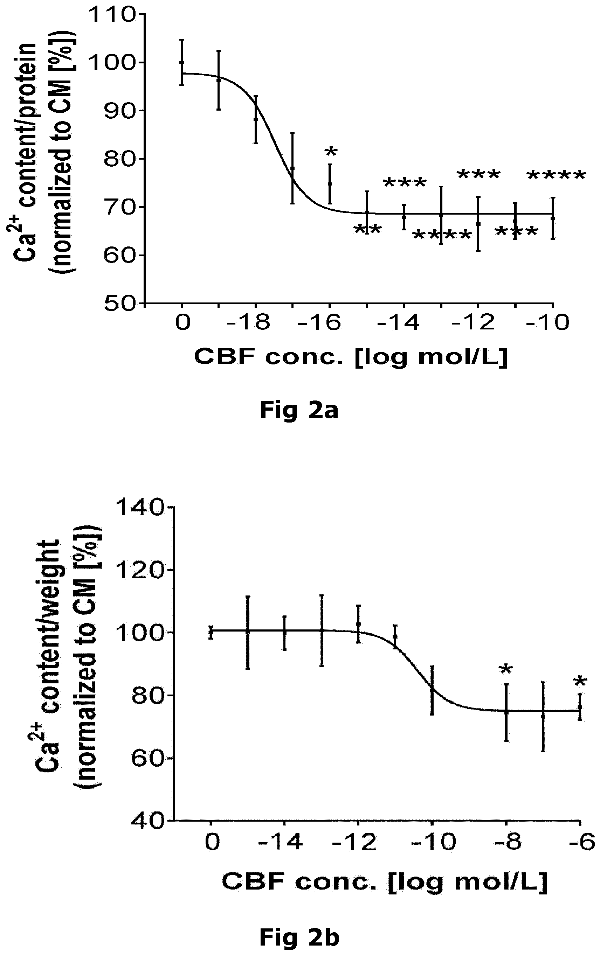

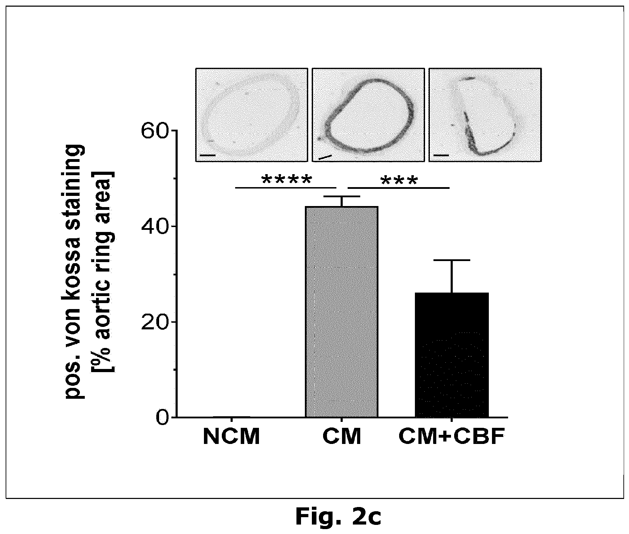

Figures 2a, 2b and 2c represent the CBF-induced inhibition of calcification in aortic rings and vascular smooth muscle cells, as described in example 2. Figure 2a represents the Dose-response effect of CBF on Ca²⁺ content of cultivated human aortic smooth muscle cells. Bonferroni's multiple comparisons were used as a post-test (N=10 per group). Figure 2b represents the dose-response effect of CBF on Ca²⁺ content of isolated aortic rings. Data shown as mean ± SEM. *P<0.05 compared to calcifying condition in the absence of CBF based on one-way ANOVA. Bonferroni's multiple comparisons were used as a post-test. Figure 2c represents the quantification of von Kossa stained aortic rings incubated in non-calcifying conditions (non-calcifying medium (NCM), under calcifying conditions (calcifying medium (CM)) in the absence or presence of CBF (100nM), respectively. CBF significantly decreased the calcified areas of aortic rings by 40%.

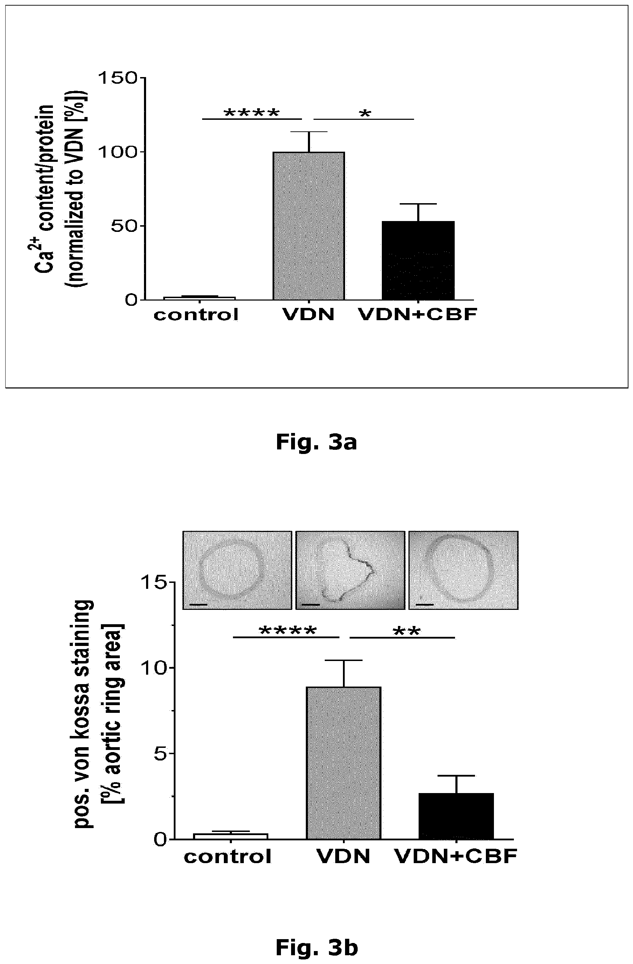

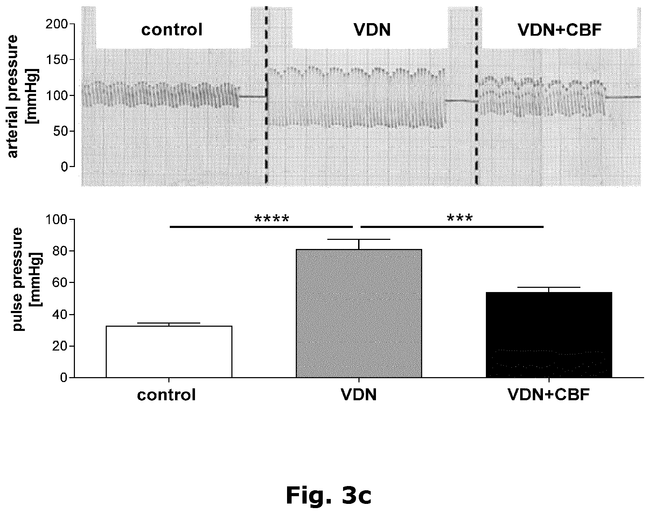

Figures 3a, 3b and 3c represent the CBF inhibition of calcification and influence on pulse pressure *in vivo,* as described in example 2. Figure 3a shows calcium content in aorta of untreated control Wistar rats and of VDN rats, either untreated (VDN) or chronically treated with CBF (31µg kg⁻¹ per day for 4 weeks). (VDN+CBF). Figure 3b shows the quantification of von Kossa stained aortic rings of rats from each experimental group: control, VDN and VDN+CBF. Figure 3c shows that CBF prominently prevents the VDN-induced rise in pulse pressure. Representative graphs of carotid arterial pressure recorded in a rat from each experimental group: control, VDN, VDN+CBF. The graph represents the mean ± SEM of pulse pressure values. Pulse pressure was increased 2-fold in VDN rats. CBF counteracted the changes in both systolic and diastolic pressure associated with VDN and reduced the pulse pressure by 63%.

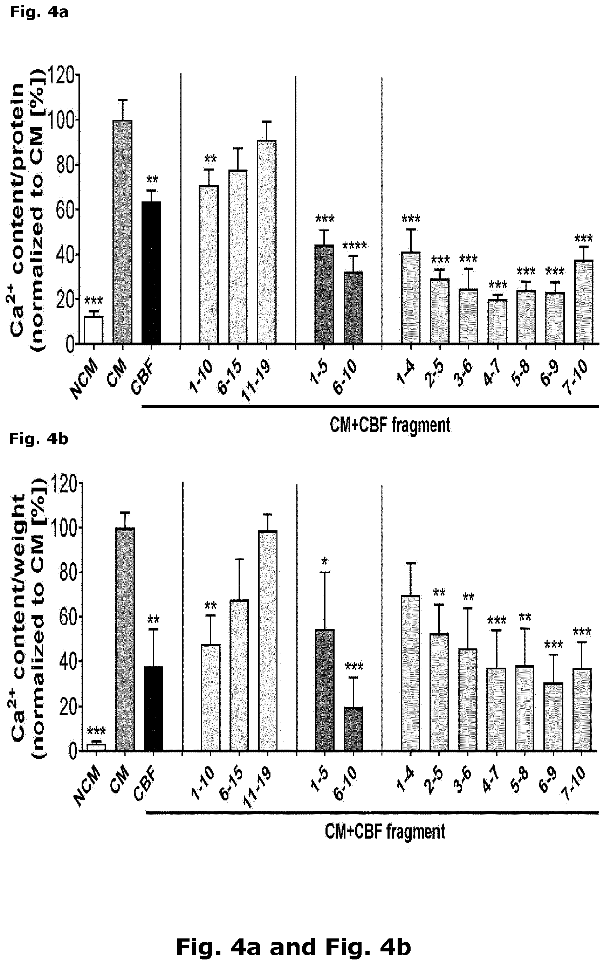

Figures 4a and 4b represent the identification of essential amino acid residues of CBF causing the calcification inhibitory effect, as described in example 3. Figure 4a shows quantification of Ca²⁺ content of cultivated human aortic smooth muscle cells (hAoSMCs) incubated in non-calcifying conditions (non-calcifying medium (NCM)) and under calcifying conditions (calcifying medium (CM)) in the absence or presence of CBF (CBF) or CBF fragments (designated by amino acid position in CBF) (100 nmol L⁻¹). Figure 4b shows the quantification of Ca²⁺ content of cultivated rat aortic rings incubated in non-calcifying conditions (non-calcifying medium (NCM)) and under calcifying conditions (calcifying medium (CM)) in the absence or presence of CBF or CBF fragments (100 nmol L⁻¹).

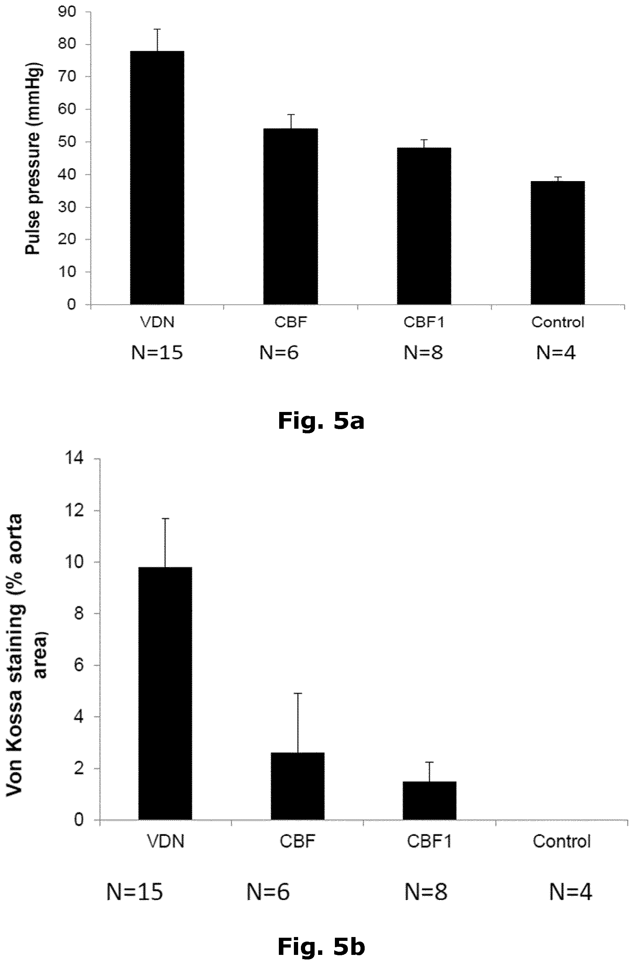

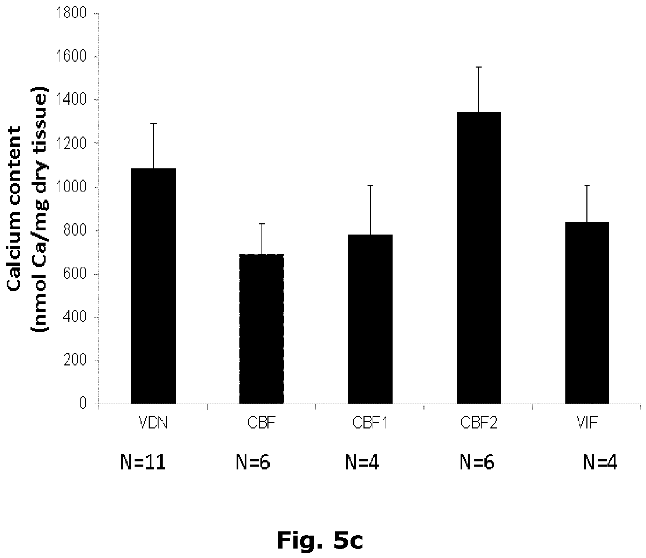

Figures 5a, 5b and 5c represents the *in vivo* effect of CBF (SEQ ID N°1), or CBF1 (SEQ ID N°2) on pulse pressure (Fig. 5a), Von Kossa aorta staining (Fig 5b) and calcium dosage (Fig. 5c) in a VDN animal model, as described in example 3.

### EXAMPLES

### Example 1: Identification and characterisation of Calcium Blocking Factor

### 1.1. Material and Methods

Chemicals: HPLC water (gradient grade), acetonitrile (ACN), magnesium chloride-hexahydrate and disodium hydrogen phosphate (Na₂HPO₄) were purchased from Merck (Darmstadt, Germany). Sodium dihydrogen phosphate (NaH₂PO₄) and sodium hydroxide (NaOH) were purchased from Roth (Karlsruhe, Germany). All other substances were obtained from Sigma-Aldrich (Munich, Germany).

Mechanical disintegration and extraction: Fresh bovine adrenal glands were obtained on ice from a local slaughterhouse, and the lipid matter was removed. Adrenal glands were then cut into pieces of about 1 g snap-frozen in liquid nitrogen for 30 min and stored at -80°C for 12 h followed by lyophilisation. The freeze-dried adrenal glands were then mechanically pulverized at 4°C. Seventy ml of 0.6 mmol L⁻¹ perchloric acid (T=4°C) was added to 700 g of pulverized adrenal glands. The resulting mixture was homogenised with an Ultra-Turrax (9.500 g min⁻¹) for 1 min at 4°C. This procedure was repeated 10 times. The homogenate was centrifuged at 2000 g for 15 min at 4°C. The supernatant's pH was adjusted to 9.0 - 11.0 by adding an aqueous saturated KOH solution. The supernatant was stored at -20°C for at least 12 h to precipitate KClO₄.

Preparative reversed-phase chromatography: The supernatant of the homogenate was adjusted to pH 6.5 by 0.1 mol L⁻¹ HCI, and the samples were loaded onto a reversed-phase chromatography column (LiChroprep RP C 18, 310 x 25 mm, Merck, Darmstadt, Germany). The chromatographic step was performed on a BioRad BioLogic DuoFlow HPLC device with UV-detection at 280 nm; 0.1% trifluoroacetic acid (TFA) in water was used as eluent A and 80% acetonitrile in water was used as eluent B with a flow rate of 3 ml min⁻¹. A stepwise gradient (20, 40, 60, 80, and 100% eluent B) was used to elute the retained substances from the chromatographic column. The fractions were lyophilized and then resuspended in 20 mmol L⁻¹ K₂HPO₄ in water.

Effect of chromatographic fractions on *ex vivo* aortic vascular calcification: Aortas from adult male Wistar rats were gently dissected and divided into segments (length: 3-4 mm). The aortic segments were incubated with calcifying medium (Dulbecco's modified Eagle's medium containing 2.8 mmol L⁻¹ phosphate) in the presence of chromatographic fractions of the adrenal glands. The aortic rings were cultured for 7 days at 37°C in a humidified atmosphere containing 5% CO₂ and media was changed every two days. Calcium content was determined as described below.

Anion-exchange chromatography: the lyophilized fractions were resuspended in 20 mmol L⁻¹ K₂HPO₄ in water, and the pH was adjusted to 8 with 0.1 mol L⁻¹ NaOH or 0.1 mol L⁻¹ HCl. The eluate was further fractionated by preparative anion exchange chromatography (column: Superformance^{™} 16, 150 ml, 30 cm x 1.6 cm, gel: Fractogel EMP TMAE^{™}; both Merck, Darmstadt, Germany). Twenty mmol L⁻¹ K₂HPO₄ in water was used as eluent A; 1 mol L⁻¹ NaCl in K₂HPO₄ (20 mmol L⁻¹ in water) was used as eluent B with a flow rate of 1 ml min⁻¹. The retained substances were eluted from the chromatographic column using a stepwise gradient (20, 40, 60, 80, and 100% eluent B), with detection at 280 nm.

Reversed-phase chromatography: the eluate from the anion exchange column was adjusted to pH 6 with 0.1 mol L⁻¹ NaOH or by 0.1 mol L⁻¹ HCl. One hundred µl of 1 mol L⁻¹ trifluoroacetic acid (TFA) was added to each fraction of the anion exchange eluate. Thereafter, the samples were loaded onto a reversed-phase chromatographic column (LiChroprep RP C 18, 310 x 25 mm, Merck, Darmstadt, Germany); 0.1% TFA in water (1:1000, v/v; eluent A) was used for equilibration (flow rate: 3 ml min⁻¹), and 80% acetonitrile in water (80:20, v/v; eluent B) and a stepwise gradient using 20, 40, 60, 80, and 100% eluent B was used for elution (flow rate: 3 ml min⁻¹). The eluate was lyophilized and stored at -20°C until further fractionation. Thereafter, the lyophilized fractions were resuspended in 15 ml H₂O and were fractionated by a reversed-phase chromatographic column (LiChroprep RP C 18e, 100 x 4.6 mm, Merck, Darmstadt, Germany). Once again, 0.1% trifluoroacetic acid (TFA) in water (1:1000, v/v) was used as eluent A and 80% acetonitrile in water (80:20, v/v) as eluent B; a linear gradient (1% to 100% ACN in 90 min; flow rate: 1 ml min⁻¹) was used for fractionation.

The chromatographic purification was performed on a BioLogic DuoFlow HPLC-device (Biorad, Munich, Germany) with a UV-detector at 280 nm. The eluate fractions were lyophilized and were resuspended in 10 µl H₂O. Each fraction of the chromatographic procedure was again screened for inhibitory effects on calcification by using aortic culture and a calcification assay.

Matrix-assisted laser desorption/ionization mass-spectrometry: The lyophilized fractions from the reversed-phase chromatography were analyzed by matrix-assisted laser desorption/ionization mass-spectrometry (MALDI-MS) and MALDI-TOF/TOF fragment ion analysis. The lyophilized fractions were resuspended in 10 µL H₂O. One µL of each fraction was prepared on a prestructured MALDI sample support (MTP AnchorChip^{™} 400/384, Bruker Daltonics, Bremen, Germany) using 2,5-dihydroxybenzoic acid (DHB) affinity sample preparation ⁴¹. Mass-spectrometric measurements were performed on a Bruker Ultraflex-III TOF/TOF instrument (Bruker-Daltonics, Bremen, Germany). The instrument was equipped with a Smart beam^{™} laser operating with a repetition-rate of 100-200 Hz. On average, the presented spectra represent the sum of 200 single-shot spectra for MS mode, and 600 for MS/MS mode. Mass-spectra of positively charged ions were analyzed in the reflector mode using delayed ion extraction. Fragment ion spectra were recorded using the LIFT option of the instrument. The calibration constants were determined using standard peptides prepared on positions adjacent to the sample, resulting in an error of <100 ppm for the recorded mass-spectra. Peptide identification using the obtained fragment ion mass data was performed using the Mascot search engine (Matrix Science, London, UK) as well as the RapideNovo 3.0.1 sequencing Tool (Bruker-Daltronic, Bremen, Germany).

Electrospray-ionization-mass-spectrometry (ESI-MS): The MS/MS fragmentation analysis was also performed by liquid-chromatography/ electrospray-mass-spectrometry (ESI). Two capillary HPLC-pumps (G1376A, Agilent, Böblingen, Germany) with micro vacuum degasser (G1379B, Agilent, Böblingen, Germany), an automatic micro-well-plate autosampler (G1377A, Agilent, Böblingen, Germany), and a diode array multi-wavelength detector (G1365D Agilent, Böblingen, Germany) were used for chromatography. Chromatography was carried out using an Agilent Zorbax SB-C18 column (5 µm, 150 x 0.5 mm, Agilent, Böblingen, Germany), the column temperature was adjusted at 25°C, and 0.01% TFA and 0.1% formic acid (FA) in water was used as eluent A with 98% acetonitrile in water used as eluent B in an injection volume of 5 µL. A flow rate of 10 µL min⁻¹ and a linear gradient (0 min: 1% eluent B; 30 min: 60% eluent B) were used for elution of retained substances from the column. The ESI-mass-spectrometry was performed in enhanced positive mode. The range of m/z was 80 to 2800. The scan speed of measured mass-spectra was 26,000 m/z per second. The accumulation time was adjusted to 200 ms. The flow rate of the nitrogen gas was 5 L min⁻¹. The temperature of the transfer-capillary was adjusted to 300°C with the capillary voltage at 1,500 to 4,500 V. The mass-spectrometric data were accumulated by the HyStar software 3.2 (Bruker-Daltonics, Bremen, Germany) and analyzed by Data Analysis 4.0 (Bruker-Daltonics, Bremen, Germany). Glutathione was used as an internal standard for calculation of the recovery rate.

Peptide synthesis: "Calcification blocking factor" (CBF) was synthesized automatically by the solid-phase method using standard Fmoc chemistry in continuous flow mode [TentaGel S Random-Access Memory (RAM) resin 0.21 mmol g⁻¹ for peptide amides, TentaGel S p-hydroxybenzoic acid (PHB) resin (Rapp Polymere, Tuebingen, Germany) for the free acid of urocortin, o-benzotriazole-N,N,N',N'-tetramethyl- uronium-hexafluoro-phosphate (HBTU), 2 equivalents of N,N-diisopropylethylamine (DIEA), coupling for 20 min, deblocking with 20% piperidine in N,N-dimethyl formamide (DMF) for 15 min, and final cleavage with 95% TFA/5% water for 3 h. Purification of the crude peptide was carried out by preparative HPLC on PolyEncap A300 (10 µm particle size, 250 mm x 20 mm, Bischoff Analysentechnik, Leonberg, Germany) in water with increasing concentrations of ACN as mobile phase. An eluent gradient of 5-70 (v/v-%) ACN/water (0.1% TFA) over 70 min with a flow rate of 10 ml min⁻¹ was used. The purified peptide was lyophilized. The peptide was characterized by MALDI mass-spectroscopy on a Voyager-DE STR BioSpectrometry Workstation MALDI-TOF mass-spectrometer (Perseptive Biosystems, Framingham, US) using R-cyano-4-hydroxycinnamic acid and sinapinic acid as matrix, and gave the expected (M + H)⁺ mass ⁴².

Statistics: Statistical analysis was performed using GraphPad Prism 8 (GraphPad Software, USA), and the data were represented as mean ± SEM. Analysis of variance (ANOVA) for a single factor (one-way) or for multiple factors (two-way) was performed to determine differences between treatment groups and the control group. In both, Bonferroni's multiple comparisons were used as a post-test. Differences at P<0.05, *P<0.05, **P≤0.01, ***P≤0.001, ****P≤0.0001 were considered to be statistically.

### 1.2. Results

The extracts of bovine adrenal glands were desalted, concentrated, and fractionated by preparative reversed-phase chromatography. Each fraction of the reversed-phase chromatography was tested for an inhibitory effect on VC using the *ex vivo* aortic ring calcification assay and then further fractionated by anion-exchange chromatography. Fractions that showed inhibitory activity were further purified by reversed-phase chromatography. The resulting fractions were again tested for inhibitory effects on VC. A characteristic reversed-phase chromatogram is shown in Fig. 1a. The fractions showing strong inhibitory effects on VC (shown by the arrow in Fig. 1a) were analyzed by mass-spectrometry. The mass-spectrum of one of these fractions showed an intense signal peak with a molecular mass of 2,297 Da (M+H⁺) (Fig. 1b). This peptide was further fragmented and sequenced using MALDI-TOF/TOF mass-spectrometry and identified using a Mascot search engine as LEGEEEEEEDPDRSMRLSF. This amino-acid sequence corresponds to the amino-acids 300-318 of bovine chromogranin A.

### 1.3. Conclusion

To test whether CBF is a component of human plasma, the purification procedure led to the isolation a peptide with molecular mass (m/z) of 2,243 (M+H⁺) from plasma. The amino-acid sequence of the underlying peptide was identified as LEGQEEEEDNRDSSMKLSF (SEQ ID N°1), and corresponded to the amino-acid sequence of the human CBF peptide from human chromogranin A. To validate the amino-acid sequence, MS/MS fragments spectra of the authentic peptide with the amino-acid sequence LEGQEEEEDNRDSSMKLSF and the isolated peptide were compared revealing identity of both peptides.

### Example 2: Inhibition of calcification in aortic rings and vascular smooth muscle cells by CBF, and influence of CBF on pulse pressure in vivo

### 2.1. Material and methods

Cell culture and calcification induction in human aortic smooth muscle cells: Human aortic smooth muscle cells (hAoSMCs) (Promocell, Heidelberg, Germany) were cultivated in smooth muscle cell medium (Promocell, Heidelberg, Germany). The hAoSMCs were seeded in 48 well plates (5x 10³ cells/well) at 80% confluence. Dulbecco's modified Eagle's medium (DMEM) containing 25 mmol L⁻¹ glucose supplemented with 2.5% fetal calf serum and 2.8 mmol L⁻¹ phosphate were used to induce calcification (calcifying medium, CM). The calcifying medium was supplemented with CBF (max. concentration 100 pmol L⁻¹) or CBF fragments (100 nmol L⁻¹). As reference medium, DMEM containing 25 mmol L⁻¹ glucose supplemented with 2.5% fetal calf serum and 0.9 mmol L⁻¹ phosphate were used (non-calcifying medium, NCM). The hAoSMCs were incubated for 7 days while the incubation medium was replaced after 48 h.

*Ex vivo* and calcification of rat aortic rings: The thoracic aorta of 8-13 weeks old male Wistar-rats were gently dissected and were cut in 3-4 mm segments. The endothelial layer of the aortic ring was manually damaged by scratching using a conventional pipette tip. The aortic rings were incubated with DMEM containing 25 mmol L⁻¹ glucose supplemented with 2.5% fetal calf serum and 2.8 mmol L⁻¹ phosphate to induce calcification. The calcification medium was supplemented with CBF (max. concentration 1 µmol L⁻¹) or CBF fragments (100 nmol L⁻¹). As reference medium, DMEM containing 25 mmol L⁻¹ glucose supplemented with 2.5% fetal calf serum and 0.9 mmol L⁻¹ phosphate were used (non-calcifying medium, NCM). The aortic rings were incubated for 7 days while the medium was replaced after 48 h. Effect of CBF in an animal model with enhanced arterial calcification: An animal model of elastocalcinosis with proven increased arterial calcification (Niederhoffer et al., "Aortic calcification produced by vitmin D3 plus nicotine", J. Vasc. Res. 34, 386-98, 1997) was chosen to assay the effects of CBF *in vivo.* Animal experiments were approved by the regional ethics committee "Comité d'éthique pour l'expérimentation animale Languedoc Roussillon N°36", with the agreement number APAFIS 2018072715539434#17270v4, and conformed to the Guide for the Care and Use of Laboratory Animals, NIH (National Academies Press US, 8th edition, 2011). Three groups of 6 weeks old Wistar rats (Charles River Laboratories, L'Arbresle, France) were given a regular rat chow (A04, Safe, Villemoisson-sur-Orge, France) and spring water (Mont Roucous^{®}, France; calcium 60 µM) *ad libitum* for one week before experiments. Two groups received CBF (31 µg kg⁻¹ per day) or its vehicle (saline) infused through an osmotic pump (Alzet 2004, Charles River, France) implanted under the skin. CBF treatment was initiated 3 or 4 days before induction of elastocalcinosis (vitamin D₃ plus nicotine, or VDN rats) with a single injection of vitamin D₃ (300,000 IU kg⁻¹, i.m.) and two gavages of nicotine (25 mg kg⁻¹, 5 ml kg⁻¹) on the same day, as previously described ⁴⁷. Untreated rats served as control rats (sham operation and saline administration). Four weeks later, rats were anaesthetized (ketamine-xylazine), and a catheter was inserted into the right carotid artery for blood pressure measurement. After 10-15 min of equilibration, systolic, diastolic, mean arterial, and pulse (systolic-diastolic) pressures were determined. The aorta was gently dissected, adventitial fat removed, and cut into segments (3-4 mm) for further analysis.

Histological calcification staining and immunofluorescence staining: Aortic rings were placed in 1 ml of 4% formaldehyde for 1 h to prepare for histological and immunohistochemical staining. The rings were dehydrated and embedded in paraffin and 5 µm slices were prepared by a microtome (Leica RM 2250, Wetzlar, Germany). The slices were placed on a glass slide, deparaffinized in xylene, and dehydrated in decreasingly concentrated sopropanol solutions before staining. Von Kossa staining was performed to visualize calcified areas of the aortic rings. The aortic ring slides were incubated with 5% silver nitrate in the presence of UV light at 405 nm for 1 h, washed three times with double-distilled water (ddH₂O), incubated in the presence of 5% sodium thiosulfate in water for 1 min, and washed three times with ddH₂O. Next, nuclear fast red (Merck; Darmstadt, Germany) was added to the slides for 5 min. The slides were then rinsed, dehydrated with ethanol, incubated for 5 min in xylol, and covered with Vitro-Clud (R. Langenbrinck; Emmendingen, Germany). For immunohistochemical analyses, sections were blocked with blocking solution (1% bovine serum albumin (BSA)) for 30 min at room temperature. The sections were incubated with monoclonal mouse anti-α-SMA antibody 1:200 (Dako, Waldbronn, Germany), polyclonal rabbit anti-phospho-SMAD1 antibody 1:100 (Biorbyt, Cambridge, UK), or polyclonal rabbit anti-phospho-SMAD5 antibody 1:200 (Biorbyt, Cambridge, UK) at 4°C overnight. Detection was carried out for α-SMA staining with polyclonal goat anti-mouse-Cy3, 1:300 1 h, at room temperature (Jackson ImmonoResearch, Cambridgeshire, UK) and for p-SMAD1 and p-SMAD5 staining with polyclonal goat anti-rabbit-Biotin (Vector Laboratories, Burlingame, CA, USA), 1:100 for 30 min at room temperature, followed by Strepavidin-FITC (Vector Laboratories, Burlingame, CA, USA), 1:30 for 30 min at room temperature. Samples were visualized with a LEICA DM5500B microscope equipped with a digital imaging system. Staining or protein expression was determined by the area stained and expressed as a percentage of the total aortic area section, using ImageJ software (National Institutes of Health, USA) after RGB split and segmentation of the image.

Measurement of calcium content: For determination of calcium content, cells or aortic rings were washed 3 times with PBS without calcium. Aortic rings were dried and weighed. Dried aortic rings and cells were decalcified with 0.1 M HCI for 24 h, and the calcium content in the supernatant was determined by the o-cresolphthalein complexone method (Clinical Chemistry Assay Kit/Calcium, County Antrim, UK) according to the manufacturer's protocol. Cell content was extracted in 0.1 M NaOH 0.1% sodium dodecyl sulfate (SDS), and protein content was measured by the bicinchoninic acid (BCA) protein assay method (Thermo Fisher Scientific, Germany). The calcium content was normalized to total protein for cells or dry weight for aortic rings.

### 2.2. Results

The effects of synthetic human CBF peptide on calcification of human aortic smooth muscle cell (hAoSMC) *in vitro* was investigated. The threshold concentration of CBF for decreasing calcification in hAoSMCs was in the range of 1 amol L⁻¹, and the corresponding EC₅₀ was in the range of 10 amol L⁻¹ (Fig. 2a). Afterwards, the effects of human CBF peptide on calcification of rat aortic rings *ex vivo* have been validated by culturing aortic rings in calcifying medium (CM) in the presence of increasing CBF concentrations. The calcification was reduced by CBF in a dose-dependent manner as shown by Ca content quantification (Fig. 2b). The threshold concentration of CBF was in the range of 0.01 nmol L⁻¹, and the corresponding EC₅₀ value was in the range of 100 pmol L⁻¹. Furthermore, reduced aortic ring calcification in the presence of CBF was confirmed by von Kossa staining, revealing a reduction in the calcified area by 40% (Fig. 2c).

Next, the impact of CBF on the *in vivo* calcification process was analyzed using the VDN animal model. Continuous CBF treatments via an osmotic pump for 4 weeks significantly reduced the Ca²⁺ content in vessels of treated rats compared to the untreated animals (Fig. 3a). Aortic calcification was visualised by von Kossa staining of aortic segments from control (left), untreated (middle), and CBF-treated (right) male VDN rats. CBF treatment significantly decreased the calcified surface by 70% (Fig. 3b). In addition, chronic treatment with CBF *in vivo* caused a significant decrease in the arterial pressure in the VDN animals and pulse pressure decreased from 81 ± 6 to 53 ± 3 (P≤0.001) (Fig. 3c). Although pulse pressure was increased 2-fold in VDN rats, CBF counteracted the changes in both systolic and diastolic pressure associated with VDN and reduced the pulse pressure by 63%.

### 2.3. Conclusion

The *in vivo* relevance of CBF for vascular calcification processes was demonstrated using a preclinical animal model of elastocalcinosis. The animals of the placebo-treated group showed more vascular calcification than the animals continually treated with CBF via a mini-pump approach, clearly demonstrating the impact of *in vivo* administration of CBF. The effect of CBF was shown by quantification of the vascular calcium content. Interestingly CBF not only reduced vascular calcium content but it also reduced pulse pressure *in vivo,* an established consequence of arterial stiffness in calcified vessels.

### Example 3: Identification of relevant amino acid sequence of CBF causing the calcification inhibitory effect

### 3.1. Results

To identify the active site of CBF, fragments of the full peptide sequence of CBF were synthesized and the inhibitory effect of the peptide fragments on calcification was analyzed both *in vitro* using hAoSMCs and *ex vivo* using aortic rings. The amino acid sequence of CBF was divided into three peptides comprising ten amino acids each. Both hAoSMCs and aortic rings were incubated in non-calcifying conditions (non-calcifying medium (NCM)) or under calcifying conditions (calcifying medium (CM)) in the absence or presence of the CBF fragment 01-10, also designated as "CBF1" (SEQ ID N°2), of the CBF fragment 06-15 (SEQ ID N°3), and of the CBF fragment 11-19, also designated as "CBF2" (SEQ ID N°4), for 7 days. The CBF fragment comprising the amino acids 01-10 significantly decreased the calcium content of hAoSMC (Fig. 4a) and aortic rings (Fig. 4b), while the CBF fragments comprising the amino acids 06-15 and 11-19 caused no significant effect. The strength of the inhibitory effect of CBF fragment 01-10 (CBF 01-10) and of the complete CBF peptide (CBF 01-19) were comparable.

Next, the inhibitory effect of the CBF 01-10 sub-fragments, designated as CBF 01-05 (SEQ ID N°5) and CBF 06-10 (SEQ ID N°6), were analyzed. Both sub-fragments shown a significant reduction of calcification in hAoSMCs (Fig. 4a) as well as in aortic rings (Fig. 4b), although the effect was stronger in cells and aortic rings incubated with the fragment CBF 06-10 (Fig. 4a and 4b). For further identification of the active segment of CBF, the following fragments were analysed: CBF 01-04 (SEQ ID N°7), CBF 02-05 (SEQ ID N°8), CBF 03-06 (SEQ ID N°9), CBF 04-07 (SEQ ID N°10), CBF 05-08 (SEQ ID N°11), CBF 06-09 (SEQ ID N°12), and CBF 07-10 (SEQ ID N°13). Each fragment showed an inhibitory effect on vascular calcification in both cells and aortic rings, with the strongest inhibition caused by CBF 04-07, CBF 05-08, and CBF 06-09 (Fig. 4a and 4b).

The peptides "CBF" (SEQ ID N°1), "CBF1" (SEQ ID N°2), CBF2" (SEQ ID N°4) and "VIF" (Vasoconstriction Inhibiting Factor) were compared for *in vivo* effect on vascular calcification, as evaluated through pulse pressure (Fig. 5a), Von Kossa staining (Fig. 5b) and calcium dosage (Fig. 5c) in a VDN animal model.

Result show that VDN animals treated with CBF had a decrease in calcium content and a decreased calcified surface of the aortas and a significantly lower pulse pressure than de control animals. VDN animals treated with CBF1 had an even more pronounced decrease in calcium content and calcified surface of the aortas as well as in pulse pressure. Treatment of VDN rats with CBF2 had no effect on these three vascular calcification - related findings. These results suggest that the active part of CBF is likely to be among amino acids 1 and 10. The results observed in the VDN animals treated with VIF did not reach statistical significance level.

### 3.2. Conclusion

For the development of clinical applications, the knowledge of the active sites of CBF is essential. This knowledge might provide the basis for exploring new approaches to prevent and treat vascular calcification in CKD patients as well as more widely in ageing populations, known to be caused by the disease. The inventors were able to demonstrate that the amino acids located between the second and ninth amino acid of CBF (representing the amino acid sequence EGQEEEED) are extremely important for the inhibitory effect on vascular calcification *in vitro* as well as *ex vivo.* This amino acid sequence contains the amino acids glutamine, glutamic acid, glycine and aspartic acid, wherein glutamine, glycine and aspartic acid occur once, and glutamic acid occurs five times. Interestingly, glutamine and glutamic acid are adjacent or separated by glycine. A carboxylic acid next to a primary amide or two carboxylic acids are likely essential for the inhibitory effect of the CBF and/or its fragments. A direct binding of calcium to the amino acid motif might be not the primary effect of CBF, since the calcium concentration clearly exceeds the CBF concentration in the media. A direct binding would not significantly affect the calcium concentration.

### Example 4: Clinical relevance of CBF

### 4.1. Results

The concentration of CBF in the plasma of healthy subjects was quantified using a mass-spectrometry-based technique as described above. The CBF plasma concentration was in the range of 266.1 ± 56.4 nmol L⁻¹ in healthy control subjects (N = 13).

To validate the relevance of CBF on human pathophysiology, the concentration of this peptide was assessed in CKD patients suffering from increased vascular calcification. The CBF concentration is significantly decreased in chronic renal failure patients (102.4 ± 23.0 nmol L⁻¹, p<0.05; N = 17) compared to healthy controls (*data not shown*)*.* CBF decrease, most likely caused by a modification of the enzyme activity of calpain 1 and/or kallikrein may have an impact on the increased vascular calcification rate in these patients.

### 4.2. Conclusion

The plasma concentration of CBF in CKD patients might be influenced by enzyme inhibition by uremic toxins and post-translational modifications of the respective enzymes, respectively, in patients suffering from declined renal function. To evaluate the relevance of the findings in humans, the plasma levels of CBF in healthy control subjects and in patients suffering from increased vascular calcification were quantified. Since no appropriate antibody for CBF is available, a mass spectrometric-based approach was used for quantification. In healthy subjects, plasma concentrations of CBF are in a range in which the peptide exhibits significant inhibitory effects on vascular calcification in our studies. While the plasma concentration is higher than the EC₅₀ value of the dose response curve, the tissue levels of CBF are likely lower than the plasma level owning to several factors, such as the endothelial barrier for CBF, enzymatic degradation, binding, or other effects. The decrease in CBF observed in CKD patients compared to controls suggests that a CBF deficit participates in increasing VC processes commonly observed in CKD patients.

## Claims

1. Peptide comprising, or consisting of, a sequence of at least 3 amino acids from SEQ ID N°2, for its use as a medicament.

2. Peptide according to any of the preceding claims, wherein said sequence of at least 3 amino acids is chosen in the group consisting of: SEQ ID N°2, SEQ ID N°5, SEQ ID N°6, SEQ ID N°7, SEQ ID N°8, SEQ ID N°9, SEQ ID N°10, SEQ ID N°11, SEQ ID N°12, and SEQ ID N°13.

3. Peptide according to any of the preceding claims, wherein said sequence of amino acids is SEQ ID N°2.

4. Peptide according to claim 1, for its use as a medicament for the prevention or the treatment of a heart and blood vessel disease.

5. Peptide according to any of the preceding claims, for its use as a medicament in the prevention or the treatment of heart and blood vessel disease associated with vascular calcification.

6. Peptide according to any of the preceding claims, wherein said peptide is able *in vitro* to decrease the calcium content of aortic smooth muscle cells cultivated in a calcifying medium.

7. Peptide according to any of the preceding claims, wherein said peptide is combined or covalently associated with a vector or a carrier.

8. Peptide according to any of the preceding claims, wherein said peptide is produced by synthesis.

9. Isolated nucleic acid sequence comprising, or consisting of, a nucleotide sequence encoding for a peptide according to any of the preceding claims, for its use as a medicament.

10. Pharmaceutical composition comprising at least one peptide according to any of claims 1 to 8 or at least an isolated nucleic acid sequence according to claim 9, and a pharmaceutically acceptable excipient.

11. Use of at least one peptide according to any of claims 1 to 8 or of an isolated nucleic acid sequence according to claim 9, for the prevention or the treatment of heart and blood vessel disease, particular for the prevention or the treatment of a disease associated with vascular calcification.
